# EUROPEAN PATENT SPECIFICATION

(11) **EP 3 904 167 B1**
(45) Date of publication and mention of the grant of the patent: **19.04.2023**
(21) Application number: 20382350.5
(22) Date of filing: 29.04.2020
(51) Int. Cl.: B60S 3/04

(54) **WASHING CABIN FOR TWO-WHEELED VEHICLES**
WASCHKABINE FÜR ZWEIRÄDER
CABINE DE LAVAGE POUR VÉHICULES À DEUX ROUES

(43) Date of publication of application: 03.11.2021
(73) Proprietor: Motos Four Angels, S.L., 28009 Madrid (ES)
(72) Inventor: TRAVESEDO JULIÁ, Camilo, 28009 MADRID (ES); VELA PALOP, Santiago, 28009 MADRID (ES); TEIJEIRO CASTRO, Rafael, 47009 VALLADOLID (ES)
(74) Representative: Herrero & Asociados, S.L.

(56) References cited:
- WO-A1-2017/114763
- DE-A1- 19 824 271
- DE-U1- 9 112 116
- FR-A1- 2 976 539
- US-A- 5 133 375
- US-A1- 2009 217 955

## Description

### Technical field of the invention

The present invention relates, as its name indicates, to a washing cabin for two-wheeled vehicles, although it is also applicable for 3-wheeled vehicles as long as they are similar in structure to the previous ones, that is, with a structure similar to a motorcycle. Likewise, the cabin of the invention shall also be suitable for washing vehicles with or without motors such as bicycles.

The object of the present invention is, on the one hand, to increase the efficiency of existing washing devices and, on the other hand, to minimize the energy and maintenance costs of said devices. All this, while ensuring and improving the integrity of the vehicle during the washing phase.

### Background of the invention

Washing a two-wheeled vehicle is a delicate task, much more than that of a four-wheeled vehicle since there are many parts of the vehicle exposed to the action of the elements used in washing.

Thus, in general, while in a four-wheeled vehicle friction washing can be carried out with direct action of elements such as brushes, rollers, etc., in the case of a two-wheeled vehicle, motorcycles or bicycles, this is not possible.

Specifically, the two fundamental reasons why it is not possible are, on the one hand, the aforementioned fragility of two-wheeled vehicles and, on the other, their instability.

As regards the latter, it is known that in order to wash the vehicle it must be supported, since by its own construction it is not possible for the vehicle to remain stable in an equilibrium position. Although this can be done with trestles or sheep's foot, this is not enough if one acts thereon during cleaning, that is, if there are any external elements that interact with the vehicle. For example, if an automatic brush is applied directly to the vehicle, there is a high risk that the vehicle will lose stability and fall to the ground, which should obviously be avoided at all costs.

In order to try to avoid this problem, there is already some equipment or facilities for washing two-wheeled vehicles that instead of using brushes or moving elements use low pressure water jets. However, a main feature of these washing systems is movement. Most of them leave the vehicle stationary and try to wash the vehicle by moving a water jet bridge in front of the vehicle, but there are also those who leave the water jets fixed and it is the vehicle that moves.

An example of this can be seen in document FR2976539, wherein the vehicle moves back and forth in front of a system provided with water jets and that also needs a system of articulated arms that approach the vehicle to improve the incidence of the jets, since, as the equipment used is a low pressure one, this makes it difficult to wash the vehicle optimally if it is carried out at a distance. This system, however, has several important drawbacks. Specifically, it has a high mechanical complexity, which increases the risk of breakdowns and hinders its correct operation. Likewise, the high number of mechanisms also implies high energy consumption to be able to operate them all. In addition, as already noted, it has two fundamental drawbacks such as the existence of moving elements that can damage the vehicle and the fact that it is necessary to move the vehicle. The latter, in addition to increasing the risk of the vehicle being damaged, means that a large installation space is required.

Another known example is the one described in DE9112116, wherein two circular and flat pieces on which the water jets are mounted are shown. This avoids the problem of approaching moving parts closer to the vehicle, so the risk of damage disappears, but there is still considerable technical complexity and high energy consumption. Also, the vehicle moves again during its cleaning, so the problem of the space it occupies does not disappear.

Finally, document US2009217955 is also known, wherein a system is shown that eliminates the problem of space because the vehicle remains stationary and it is a single-arm bridge that moves around the vehicle. However, said mobile bridge implies both that there is a risk of contact with the vehicle and a high technical complexity due to the need of motors, guides and sensors necessary for this movement.

Document DE19824271 discloses an automatic washer for especially two-wheeled vehicles that incorporates a water spray device with two high pressure nozzles directed on the side of the bicycle etc. to be cleaned. The spray device is located in a cleaning booth and is moveable in a plane parallel to a booth side wall, so that it executes an oscillating zig-zag movement during cleaning. Due to this, the water jet generated by the nozzles continuously covers the surface of the opposite side wall. The nozzles are moved by an electric, hydraulic, or compressed air drive system. The booth contains a holder for the bicycle. Document DE 198 24 271 A1 discloses the preamble of claim 1.

Document US5133375 discloses a washer for a conventional wheelchair has a washing chamber within an enclosure with fluid-driven rotatable washer arms having suitably angles nozzles for directing cleaning and rinsing fluids onto a wheelchair mounted on a slidable rack just above the slanted drain floor of the enclosure. The device also provides for rotating the large side support wheels during the washing cycles while keeping the chair from moving.

Therefore, there are no known systems that do not have some of the aforementioned drawbacks, because although they solve the problem of washing two-wheeled vehicles to a greater or lesser extent, or they do so involving an excessive complication of mechanisms and a high energy consumption, or risk to the vehicle, or at the cost of needing a large facility space.

On the other hand, in addition to cleaning these types of vehicles by the methods outlined above, there is now an unmet need in the state of the art to go further in said cleaning and carry out disinfection capable of eliminating viruses and other pathogens. Specifically, this is especially important when cleaning two-wheeled vehicles, for example shared-use electric bicycles or motorcycles, which are used by a large number of users.

### Description of the invention

The washing cabin for two-wheeled vehicles of the invention solves the problems of the state of the art described above, since it consists of an effective washing device, simple in terms of handling and number of elements, which occupies little space and low energy consumption, which ensures the integrity of the vehicles and, in addition, offers an effective disinfection that allows the elimination of viruses and pathogens.

For this, in general, the cabin of the invention allows for non-contact, high-pressure washing, which, in addition, dispenses with moving elements that approach the vehicle, which remains stationary or fixed throughout the operation.

In addition, it should be noted, as indicated above, that the cabin of the invention is also applicable for 3-wheeled vehicles as long as they are similar in structure to two-wheeled vehicles, that is, as long as they have a structure similar to a motorcycle. Likewise, it will also be appropriate for washing vehicles with or without a motor, such as bicycle.

More specifically, the washing cabin for two-wheeled vehicles comprises at least one washing chamber where the vehicle is located and a machinery cabinet wherein the devices and mechanisms necessary for its operation and control are housed.

The washing chamber forms a closed compartment consisting of two major side walls and two minor walls, one of these smaller walls being intended to house the door for entering the interior compartment.

Inside the washing area, the washing turbines will be placed, which are responsible for propelling the water on the vehicle during the washing cycle. Thus, both the two largest side walls and the smallest side wall that does not constitute the entrance comprise washing turbines that can be controlled by sections, being able to operate all at once or by sectors depending on various washing options, features of the vehicle, etc.

Specifically, the washing turbines comprise a fixed central body connected at one end to the high-pressure hydraulic circuit and, on the other end, to a head that in turn comprises at least two tubular arms that end in two water impeller nozzles, wherein said head is a rotating head that rotates by being moved by the force of the water that passes through it.

More specifically, each of the tubular arms is formed by a first section attached to the rotary head and a second section ending with a water impeller nozzle, wherein both sections form an angle with each other.

Furthermore, the arrangement of the tubular arms is such that the axis that passes through the center of the impeller nozzles is not parallel with respect to the axis of rotation of the rotary head, that is, that both axes form an angle α with each other. Thus, this angled arrangement between the impeller nozzles causes the washing cone or water cone they project during washing to overlap, optimally covering the surface of the vehicle.

In other words, if the axis that passes through the center of the impeller nozzles remains parallel or practically parallel with respect to the axis of rotation of the rotary head, that is, the angle α is 0° or very close to 0°, this implies that the washing cones generated by each impeller nozzle overlap to a great extent, which means having to use a greater number of them or ducts to correctly cover the entire surface of the vehicle. This, obviously, is not desirable, since it not only makes the installation more expensive and complicated, but the consumption of water and energy is greater and slows down the washing process.

However, said angle α must not be excessive either since in this case the washing cones open too much, leaving washing areas uncovered and, therefore, carrying out a faulty or insufficient washing.

According to the tests and trials carried out, the separation angle α between the axes should be in the range between 2° and 20° and, optimally, between 8° and 12°.

On the other hand, as mentioned above, the machinery cabinet includes the devices and mechanisms necessary for its operation and control.

Among others, said cabinet comprises the hydraulic system, which is made up of a low pressure and a high pressure circuit. The low pressure circuit is responsible for capturing the washing water from the supply network and has two sub-circuits, one for cold water and the other for hot water. This allows washing with cold water, hot water or a mixture of cold water for some washing phases and hot water for others. In the low pressure circuit no pump is included, so it works with the pressure existing in the supply network.

Inside the hot water sub-circuit there is a boiler to heat the washing water, which is delivered to a thermo-tank that stores it to ensure its supply.

The low pressure circuit is routed through two solenoid valves, one for cold water and the other for hot water, to the high pressure pump, which is responsible for sending high pressure water to the washing turbines. Pumps supplying chemicals such as soap, waxes, polishes and any other product that is intended to be used in the wash to improve it, are connected to the inlet of the high pressure pump.

The output of the high pressure pump is directed to a high pressure manifold on which the different high pressure circuits are located. Each of the high pressure circuits is controlled by a solenoid valve and supplies fluid under pressure to a block of washing turbines that are operable as intended or according to the chosen washing program.

In this way, a sectioned washing can be carried out, supplying pressurized fluid to a few turbines instead of all those existing in the machine, which allows reducing the size of the high-pressure pump and improving the efficiency of washing by concentrating more pressure and flow for washing.

Finally, as already indicated, the invention responds to the growing need to carry out not only conventional cleaning of vehicles, but also to carry out disinfection and / or elimination of viruses and pathogens thereof.

For this, the cabin of the invention incorporates a double mechanism, which ensures this function. Specifically, the cabin of the invention is capable of comprising both an ozone dosing circuit and a set of UV ultraviolet rays lamps that will complement the cleaning of the vehicle.

Finally, either the washing sequence or the turbines to be used as well as the equipment subsystems, high and low pressure, heating, drying, disinfection, door, lighting, security and payment means are commanded and / or controlled by a control system which includes the necessary electronics and software.

### Brief description of drawings

To complement the description that is being made and in order to help a better understanding of the features of the invention, a set of drawings is included as an integral part of said description in which, by way of illustration and not limitation, the following has been represented:

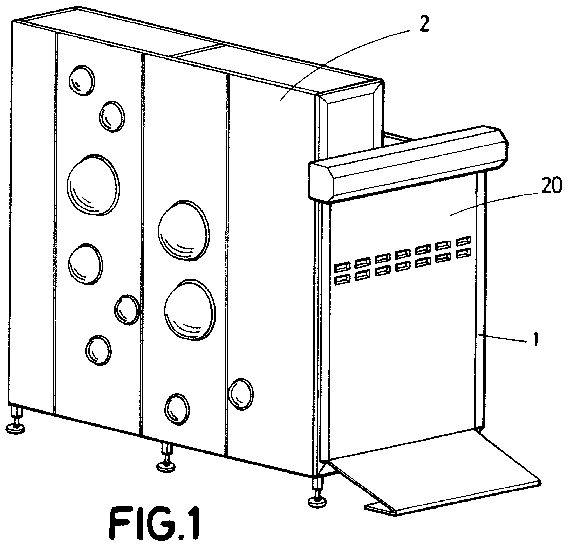

Figure 1.- Shows a perspective view of the closed washing cabin of the invention.
Figure 2.- Shows a perspective view of the washing cabin of the invention with the door open and with a vehicle inside.
Figure 3.- Shows a sectioned elevation view of the cabin of the invention, showing part of the machinery cabinet and the arrangement of the turbines on one of the side walls.
Figure 4.- Shows a sectioned plan view of the cabin of the invention, showing part of the machinery cabinet and the ground where the vehicle is located.
Figure 5.1 and 5.2.- They show respective views of the turbines, one in perspective and the other showing the hidden parts respectively.
Figure 6.- Shows a schematic view of the washing area covered by the ducts on one of the side walls.
Figure 7.- Shows, finally, a side elevation view wherein the elements of the machinery cabinet are schematically seen.

### Preferred embodiment of the invention

In view of the figures provided, it can be seen how, in a preferred embodiment, the cabin of the invention comprises a support structure (1) that is paneled on the outside by a set of external walls or panels (2) that make up the enclosure.

Likewise, it has an internal enclosure (3) that divides the interior space into two, one having greater dimensions that constitutes the washing chamber (4) itself, in which the vehicle to be cleaned is introduced through a door (20), and another one that houses the machinery cabinet (5) in which the necessary devices and mechanisms for its operation and control are housed, which will be seen later.

According to the embodiment shown in the figures, the cabin of the invention has a rectangular prism shape, but this may also be any other shape that allows vehicle accommodation inside, for example, cylindrical, semi-cylindrical, etc.

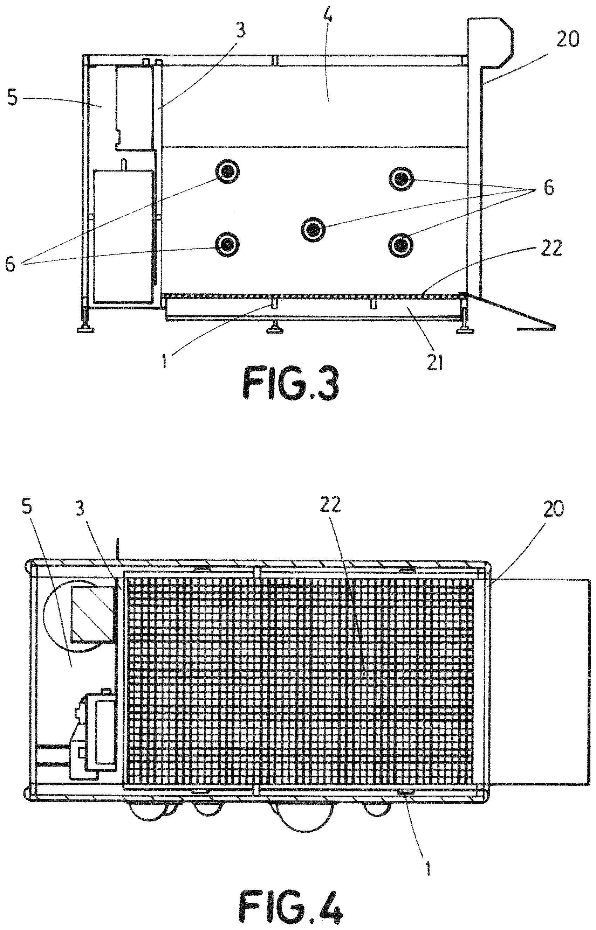

Furthermore, the cabin of the invention may comprise, according to a possible practical embodiment shown in the figures, a lower tray (21) for collecting liquids coming from the washing, which can be discharged to a water recycling equipment or directly to the drain according to its installation location. Thus, as can be seen in the figures, especially in Figure 4, a grid (22) would provide access to said lower tray (21), which would be the one on which the vehicle would be placed during cleaning.

On the other hand, according to the invention, the washing turbines (6) are arranged in the washing chamber (4), which will supply the water and the washing fluids to the vehicles.

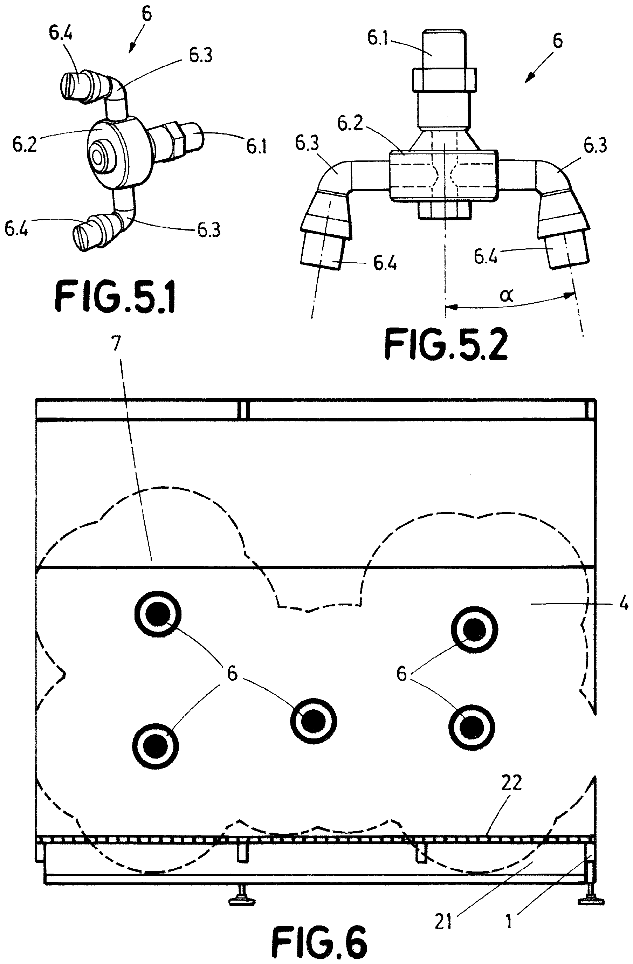

Specifically, as can be seen in Figures 5.1 and 5.2, the washing turbines (6) comprise a fixed central body (6.1) connected at one end to the high-pressure hydraulic circuit and, on the other end, to a head (6.2) that, in turn, comprises at least two tubular arms (6.3) that end in two water impeller nozzles (6.4), wherein said head (6.2) is a rotating head that rotates by being moved by the force of the water that passes through it.

More specifically, each of the tubular arms (6.3) is formed by a first section attached to the head (6.2) and a second section ending at the impeller nozzle (6.4), wherein both sections form an angle with each other.

Thus, as can be seen in the preferred embodiment shown in said figures, the arrangement of the tubular arms (6.3) is such that the axis that passes through the center of the impeller nozzles (6.4) is not parallel with respect to the axis that passes through the center of the head (6.2), which is the axis of rotation thereof, that is, that both axes, that of the impeller nozzle (6.4) and that of the head (6.2) form an angle α to each other as marked on the figure 5.2.

As explained above, said angle α must be between a minimum value such that the washing cones (7) of the impeller nozzles (6.4) do not overlap too much and a maximum value such that said washing cones (7) open too much, leaving areas of the vehicle unwashed.

According to the tests and trials carried out, these minimum and maximum values of the separation angle α between the axes must be in the range between 2° and 20° and, optimally, between 8° and 12°.

However, according to a preferred embodiment that guarantees optimal washing and, therefore, an optimal superposition or overlap of the washing cones (7) is one in which the angle α is equal to 10°.

Thus, this angled arrangement between the impeller nozzles causes the washing cone (7) or water cone that they project during washing to overlap, optimally covering the vehicle's surface.

According to the invention shown in the figures, the inclination angle α between the axes of the impeller nozzles (6.4) and that of the head (6.2) that achieves the optimal washing cone (7), is achieved by making the angle formed by the first and second section of the tubular arms (6.3) greater than 90°.

However, other alternative embodiments are not ruled out either, in which, for example, said inclination angle α between the axes of the impeller nozzles (6.4) and that of the head (6.2) is achieved because the union of the first section of the tubular arm (6.3) is attached to the head (6.2) in an inclined way.

However, in order to carry out an optimal washing, it is not only important the washing cone (7) projected by each impeller nozzle (6.4) or, in other words, the washing area covered by the washing cones (7) of each one of the impeller nozzles (6.4) comprising each wash turbine (6), but also the number and arrangement of said wash turbines (6).

Thus, according to the preferred embodiment of the invention shown in the figures in which the cabin has a rectangular prism shape, it includes, within its washing chamber (4), washing turbines (6) both on its two largest side walls and on the smaller side wall that does not constitute the entrance door (20).

More specifically, the washing turbines (6) located on the larger side walls will be responsible for cleaning the vehicle on both sides, while the washing turbines (6) located on the smaller side wall will be responsible for cleaning the front face of the motorcycle.

Likewise, another important parameter in order to achieve optimal washing of two-wheeled vehicles is the number of washing turbines (6) that will comprise each of the walls of the washing chamber (4).

According to a preferred embodiment, only partially shown in the figures, the washing chamber (4) comprises, at least:
- On the smaller side wall that is responsible for the front cleaning of the vehicle: 2 washing turbines (6).
- On each of the two largest side walls (Figure 6):
   ∘ 2 washing turbines (6) matching the area that occupies the front of the vehicle.
   ∘ A washing turbine (6) matching the area that occupies the central part of the vehicle.
   ∘ 2 washing turbines (6) matching the area occupied by the rear of the vehicle.

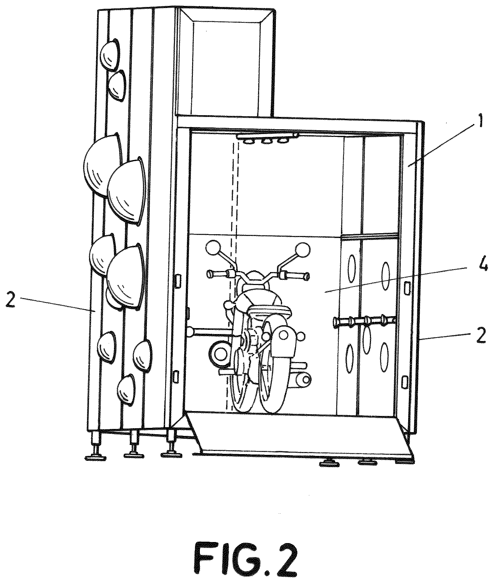

Regarding the height of the washing turbines (6) described above, for all of them the criterion that has been followed is that their washing cone (7) covers from the ground to, at least, the highest point of motorcycles that currently exist in the market, taking into account that the user, in addition, will be obliged to leave the vehicle according to a previously established or marked position inside the cabin, or at least in a very limited range of positions that allows applying the washing cones (7) correctly and so that the handlebar or front part of the motorcycle is located at the point furthest from the door, as shown in Figure 2.

Thus, in the case of those located on the smaller side wall that is responsible for the front cleaning of the vehicle, the heights of both washing turbines (6) have been calculated so that both of them jointly cover the above-mentioned area from the ground to said highest part of the motorcycles currently on the market.

In the case of those located on the two largest side walls, the same criterion has been followed regarding height, so that in the case of the front and rear ones they cover from the ground to the highest area reached by motorcycles on the market and, in the case of the central, unique one, so that it covers from the ground to the seat area, which is also perfectly typified, therefore, an additional washing turbine (6) is not necessary as in the case of the front and rear areas as explained.

However, the aforementioned rear part of the vehicles is the most critical since it is where most changes are present depending on their size, the existence of suitcases, etc. Thus, according to a possible embodiment, the two washing turbines (6) intended to wash the rear part of the vehicle may be placed, either so that the axis of the head (6.2) is substantially perpendicular thereto and in front of the axis of the wheel rear, or so that the axis of the head (6.2) is further away, behind the axis of the rear wheel and inclined with respect to the vehicle so that the projected washing cone (7) is larger and covers a greater surface area, improving the process.

On the other hand, according to a possible embodiment, it will be possible to divide into sections the action of the washing turbines (6), that is, to operate all at once or by sections depending on various washing options, vehicle features, etc. For example, the highest wash turbines (6) located matching the rear part of the vehicle may be activated for those cases in which the vehicles are large and have suitcases but being deactivated in the case of small vehicles that do not have these suitcases because in those cases the incidence of the washing cone (7) will be minimal.

Finally, according to different alternative embodiments not shown, these washing turbines (6) could also be present on the top and/or on the ground of the washing chamber (4) if an even more complete washing is to be achieved.

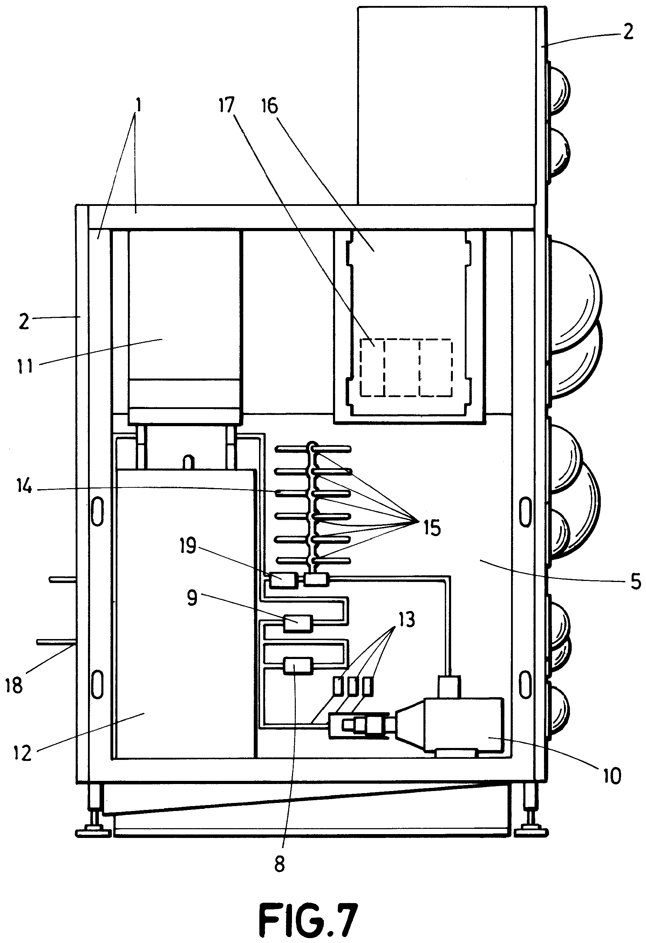

As for the different devices and mechanisms that comprise the cabin of the invention and that houses the machinery cabinet (5), according to a preferred embodiment of the invention that can be seen mainly in Figure 7, these will be as follows.

Specifically, the hydraulic system is inside the machinery cabinet (5). This is made up of a low pressure hydraulic system and a high pressure hydraulic system. The low pressure hydraulic system is responsible for supplying water to the entire cabin. It is connected to the water supply network and provides water to the two subsystems that exist within it, a cold water low pressure hydraulic subsystem and a hot water low pressure hydraulic subsystem. Each of these subsystems, cold water and hot water, has a low pressure solenoid valve (8) (9), respectively. These solenoid valves (8,9) are the ones that allow or prevent the passage of water to the high pressure pump (10).

The hot water low pressure hydraulic system comprises a boiler (11) and a thermal storage tank (12). The boiler (11) supplies hot water to the thermal storage tank (12) so that it can supply hot water to the high-pressure pump (10) when necessary, according to the washing program that is currently being executed.

In addition, the low pressure hydraulic system also includes chemical pumps (13), in charge of supplying or injecting chemical products such as soap, waxes, brightener or any other product necessary for cleaning vehicles. These chemical pumps (13) are located at the inlet of the high pressure pump (10).

On the other hand, the high pressure hydraulic system includes the high pressure pump (10), in charge of supplying the fluid for high pressure washing, for example at a pressure between 60 and 120 bar, to a high pressure manifold (14).

The high pressure solenoid valves (15) are mounted on said high pressure manifold (14). These solenoid valves (15) divide into sections the washing areas of the machine as explained above, so that it is possible to choose the areas of the vehicle that will be subjected to washing. More specifically, there is a high pressure solenoid valve (15) for each area that is intended to be sectioned.

For example, according to an example of sectioning of 6 areas as indicated above, these would be: front area, left and right front lower lateral area, left and right central lateral area, left and right rear lower lateral area, left and right front upper lateral area and left and right rear upper lateral area.

The electrical / electronic equipment of the cabin is also inside the machinery cabinet (5), which includes an electrical cabinet (16) made up of the electrical elements necessary for the operation of all the components. Furthermore, it comprises a control system such as a PLC programmable robot (17) that controls the washing sequence of the machine by means of a program recorded in its internal memory.

On the other hand, according to a possible embodiment of the invention, the machinery cabinet (5) also comprises a pressurized air inlet (18) that supplies pressurized air to the machine to carry out the drying process. This pressurized air inlet (18) is connected to a solenoid valve (19) that controls the passage of pressurized air. The solenoid valve (19) is connected to the high pressure manifold (14) and, in the drying phases, will supply air at a normal pressure of, for example, 8 bar, which, leaving the washing turbines (6), will carry out the drying of the vehicle.

Finally, as initially stated, the cabin of the invention comprises, according to a possible embodiment, means of disinfection and / or elimination of viruses and pathogens of the vehicle.

Said disinfection means can also be double, to ensure optimum disinfection.

Specifically, on the one hand the cabin of the invention comprises an ozone dosing circuit, not shown in the figures, and which may be made either independently or may use the elements that the cabin already has to carry out drying. In other words, an ozone generator included in the machinery cabinet (5) could also be connected to the aforementioned pressurized air inlet (18) to supply said ozone, either alone or in combination with the drying air in the washing chamber (4).

Specifically, according to a possible embodiment of the invention, the machinery cabinet (5) also includes an ozone generator connected to the pressurized air inlet (18), which in turn is connected to a solenoid valve (19) that controls the passage of said ozone. All this, commanded by the control system in such a way that the disinfection can be activated or not at the user's choice, even as an alternative to conventional washing, that is, without this having occurred or at the end as a complement thereto.

Furthermore, according to another possible alternative embodiment, either independently or in combination with the ozone circuit, the cabin of the invention comprises a set of ultraviolet or UV lamps located inside the washing chamber (4) and arranged so that all parts of the vehicle are irradiated, thus allowing additional disinfection. These lamps will also be commanded and / or controlled by the control system so that their activation is possible at the most favorable moment or, at the user's choice, even as an alternative to conventional washing and / or to the use of ozone, that is to say, independently of them or at the end as a complement to them.

Having said all of the above, an example of the operation of the cabin of the invention could be the one described below.

Once the vehicle to be cleaned has been introduced into the washing chamber (4), the system will be activated by means of the PLC (17). It will close the door (20) to prevent the water from escaping to the outside and so that all the water that drains during the wash falls into the collection tray (21) through the grid (22).

The water from the network enters the machine and reaches, on the one hand, the cold water low-pressure solenoid valve (8) and, on the other hand, the boiler (11). The boiler (11) heats water and stores it in the thermal storage tank (12). The hot water comes out of this and reaches the hot water low pressure solenoid valve (9). Depending on the program chosen by the user, the passage of the cold water low pressure solenoid valve (8) will open if it is in a cold water washing phase, or the passage of the hot water low pressure solenoid valve (9) will open if it is in a hot water washing phase. Thus, the solenoid valve (8,9) that has been activated, will let the water pass, which will go to the high pressure pump (10). On its way, it will come across with the chemical pumps (13), which will supply the products directly into the water flow that the solenoid valve (8, 9) may let pass through. These will supply product according to what is indicated in the washing program. Once the chemicals are included in the water, it will reach the high pressure pump (10). This pump takes the water at low pressure and raises it up to a pressure between, for example, 60 and 120 bar.

Once the water is at high pressure it leaves the pump towards the high pressure manifold (14). The washing program will indicate an opening sequence of the solenoid valves (15) so that the cycle allows for a vehicle washing by sections.

Finally, the high-pressure solenoid valves (15), when activated, send the high-pressure water to the washing turbines (6), passing through the fixed central body (6.1) thereof and reaching the head (6.2). The water pressure itself makes the head (6.2) rotate on its axis and the high pressure water reaches the tubular arms (6.3), which will project an optimal washing cone (7) as explained above.

The washing process will be completed by repeating the cycle described as many times as deemed appropriate, activating the high pressure solenoid valves (14) so that the entire vehicle to be washed is covered, performing this washing with hot or cold water as programmed.

Likewise, said washing process may be completed with the activation of the previously described disinfection elements such as the ozone circuit and / or the ultraviolet light lamps.

In addition to all the elements described, the hydraulic system includes safety elements not represented in the figures, such as non-return and safety valves that prevent the operation of the cabin from being inappropriate and from carrying out incomplete or faulty washing.

Finally, according to an alternative embodiment not described in the figures, the cabin of the invention could also comprise a low-pressure water circuit inside the washing chamber (4) in order to rinse the vehicle, in which case this could be arranged on the top of said washing chamber (4) and drop the water in the conventional way, by gravity. However, it would also not be ruled out, as previously stated, that the rinse system be also carried out at high pressure, so that the corresponding washing turbines (6), two of them, for example that would fulfill this function, would be located on the top.

In the same way, the cabin of the invention could also, on an alternative or complementary basis, comprise a low pressure circuit for the application of degreasing liquid, which may have dosing nozzles at certain places in the washing chamber (4), which could also be activated or not according to the type of washing program chosen by the user.

## Claims

1. - Washing cabin for two-wheeled vehicles, comprising:
- an interior enclosure (3) that divides the interior space into a washing chamber (4) and a machinery cabinet (5); and
- a door (20) for accessing the washing chamber (4);
- wherein the machinery cabinet (5) comprises a high pressure hydraulic circuit and the washing chamber (4) comprises several washing turbines (6);
- the washing cabin being **characterised in that**: the washing turbines (6) comprise a fixed central body (6.1) connected at one end to the high-pressure hydraulic circuit and, on the other, to a head (6.2) that rotates with the passage of water, which in turn comprises at least two tubular arms (6.3) ending in two water impeller nozzles (6.4), wherein the axis passing through the center of the impeller nozzles (6.4) forms with the axis of rotation of the head (6.2) an angle α in the range between 2° and 20°; and
- wherein each of the tubular arms (6.3) is formed by a first section attached to the head (6.2) and a second section ending at the impeller nozzle (6.4), wherein both sections are at an angle to each other;
- wherein the angle formed by the first and second section of the tubular arms (6.3) is greater than 90°.

2. - Washing cabin for two-wheeled vehicles according to claim 1, **characterized in that** the cabin has a rectangular prism shape and includes, within its washing chamber (4), washing turbines (6) both on its two largest side walls and on the smaller side wall that does not constitute the entrance door (20).

3. - Washing cabin for two-wheeled vehicles according to claim 2, **characterized in that** the washing chamber (4) comprises, at least:
- two washing turbines (6) on the smaller side wall for front cleaning of the vehicle; and
- on each of the two largest side walls:
- two washing turbines (6) in correspondence with the area where the front part of the vehicle is located.
- a washing turbine (6) in correspondence with the area where the central part of the vehicle is located.
- two washing turbines (6) in correspondence with the area where the rear part of the vehicle is located.

4. - Washing cabin for two-wheeled vehicles according to any of the preceding claims, **characterized in that** the machinery cabinet (5) additionally comprises a low pressure hydraulic system connected to the water supply network that provides water to a cold water low pressure hydraulic subsystem and to a hot water low pressure hydraulic subsystem, wherein each of them has, respectively, a solenoid valve (8.9) to allow or not the passage to a high pressure pump (10) belonging to the high pressure hydraulic system.

5. - Washing cabin for two-wheeled vehicles according to claim 4, **characterized in that** the high pressure pump (10) is connected to a high pressure manifold (14) that includes high pressure solenoid valves (15) connected to the washing turbines (6) and to a control system (17) such that it allows a total or sectioned operation of said washing turbines (6) so that it is possible to choose the areas of the vehicle that will be subjected to washing.

6. - Washing cabin for two-wheeled vehicles according to any of the preceding claims, **characterized in that** angle α is between 8° and 12°.

7. - Washing cabin for two-wheeled vehicles according to claim 6, **characterized in that** the angle α is of 10°.

8. - Washing cabin for two-wheeled vehicles according to any of the preceding claims, **characterized in that** the machinery cabinet (5) comprises a pressurized air inlet (18) connected to the high-pressure manifold (14) to supply drying air through the washing turbines (6).

9. - Washing cabin for two-wheeled vehicles according to claim 8, **characterized in that** the machinery cabinet (5) comprises an ozone generator connected to the pressurized air inlet (18) to supply said ozone to the washing chamber (4) through the washing turbines (6).

10. - Washing cabin for two-wheeled vehicles according to any of the preceding claims 1 to 8, **characterized in that** the machinery cabinet (5) comprises an ozone generator connected to a dosing circuit located in the washing chamber (4).

11. - Washing cabin for two-wheeled vehicles according to any of the preceding claims 1 to 8, **characterized in that** the washing chamber (4) comprises a set of ultraviolet light lamps.

12. - Washing cabin for two-wheeled vehicles according to any of the preceding claims, **characterized in that** it comprises a low-pressure water circuit located on the top of the washing chamber (4) for rinsing the vehicle.

13. Washing cabin for two-wheeled vehicles according to any of the preceding claims, **characterized in that** it comprises a low-pressure circuit for the application of degreasing liquid within the washing chamber (4).

## Patentansprüche

1. Waschkabine für zweirädrige Fahrzeuge, mit:
- einem Innengehäuse (3), das den Innenraum in eine Waschkammer (4) und einen Maschinenschrank (5) unterteilt; und
- einer Tür (20) für den Zugang zur Waschkammer (4);
- wobei der Maschinenschrank (5) einen Hochdruckhydraulikkreislauf umfasst und die Waschkammer (4) mehrere Waschturbinen (6) umfasst; wobei die Waschkabine **dadurch gekennzeichnet ist, dass**:
- die Waschturbinen (6) einen festen zentralen Körper (6.1) umfassen, der an einem Ende mit dem Hochdruckhydraulikkreislauf und an dem anderen mit einem Kopf (6.2), der sich beim Durchgang von Wasser dreht, verbunden ist, welcher wiederum mindestens zwei rohrförmige Arme (6.3) umfasst, die in zwei Wasserlaufraddüsen (6.4) enden, wobei die durch die Mitte der Laufraddüsen (6.4) gehende Achse mit der Drehachse des Kopfs (6.2) einen Winkel α im Bereich zwischen 2° und 20° bildet; und
- wobei jeder der rohrförmigen Arme (6.3) von einem ersten Abschnitt, der an dem Kopf (6.2) angebracht ist, und einem zweiten Abschnitt ausgebildet wird, der an der Laufraddüse (6.4) endet, wobei beide Abschnitte in einem Winkel zueinander stehen;
- wobei der Winkel, der durch den ersten und zweiten Abschnitt der rohrförmigen Arme (6.3) gebildet wird, größer als 90° ist.

2. Waschkabine für zweirädrige Fahrzeuge nach Anspruch 1, **dadurch gekennzeichnet, dass** die Kabine die Form eines rechteckigen Prismas hat und innerhalb ihrer Waschkammer (4) sowohl auf ihren zwei größten Seitenwänden als auch auf der kleineren Seitenwand, die nicht die Eingangstür (20) bildet, Waschturbinen (6) aufweist.

3. Waschkabine für zweirädrige Fahrzeuge nach Anspruch 2, **dadurch gekennzeichnet, dass** die Waschkammer (4) zumindest Folgendes umfasst:
- zwei Waschturbinen (6) auf der kleineren Seitenwand zur Frontreinigung des Fahrzeugs; und
- auf jeder der zwei größten Seitenwände:
- zwei Waschturbinen (6) in Übereinstimmung mit dem Bereich, wo sich der Frontteil des Fahrzeugs befindet
- eine Waschturbine (6) in Übereinstimmung mit dem Bereich, wo sich der zentrale Teil des Fahrzeugs befindet
- zwei Waschturbinen (6) in Übereinstimmung mit dem Bereich, wo sich der hintere Teil des Fahrzeugs befindet.

4. Waschkabine für zweirädrige Fahrzeuge nach einem der vorstehenden Ansprüche, **dadurch gekennzeichnet, dass** der Maschinenschrank (5) zusätzlich ein mit dem Wasserversorgungsnetz verbundenes Niederdruckhydrauliksystem umfasst, das ein Kaltwasser-Niederdruckhydraulikteilsystem und ein Heißwasser-Niederdruckhydraulikteilsystem mit Wasser versorgt, wobei jedes von ihnen jeweils ein Solenoidventil (8, 9) hat, um den Durchgang zu einer zum Hochdruckhydrauliksystem gehörenden Hochdruckpumpe (10) zu ermöglichen oder nicht.

5. Waschkabine für zweirädrige Fahrzeuge nach Anspruch 4, **dadurch gekennzeichnet, dass** die Hochdruckpumpe (10) mit einem Hochdruckverteiler (14) verbunden ist, der Hochdrucksolenoidventile (15) enthält, die derart mit den Waschturbinen (6) und mit einem Steuersystem (17) verbunden sind, dass eine vollständige oder abschnittsweise Betätigung der Waschturbinen (6) ermöglicht wird, sodass es möglich ist, die Bereiche des Fahrzeugs zu wählen, die dem Waschen unterzogen werden.

6. Waschkabine für zweirädrige Fahrzeuge nach einem der vorstehenden Ansprüche, **dadurch gekennzeichnet, dass** der Winkel α zwischen 8° und 12° liegt.

7. Waschkabine für zweirädrige Fahrzeuge nach Anspruch 6, **dadurch gekennzeichnet, dass** der Winkel α 10° beträgt.

8. Waschkabine für zweirädrige Fahrzeuge nach einem der vorstehenden Ansprüche, **dadurch gekennzeichnet, dass** der Maschinenschrank (5) einen Drucklufteinlass (18) umfasst, der mit dem Hochdruckverteiler (14) verbunden ist, um durch die Waschturbinen (6) Trockenluft zuzuführen.

9. Waschkabine für zweirädrige Fahrzeuge nach Anspruch 8, **dadurch gekennzeichnet, dass** der Maschinenschrank (5) einen Ozongenerator umfasst, der mit dem Drucklufteinlass (18) verbunden ist, um der Waschkammer (4) durch die Waschturbinen (6) Ozon zuzuführen.

10. Waschkabine für zweirädrige Fahrzeuge nach einem der vorstehenden Ansprüche 1 bis 8, **dadurch gekennzeichnet, dass** der Maschinenschrank (5) einen Ozongenerator umfasst, der mit einem Dosierkreislauf verbunden ist, der sich in der Waschkammer (4) befindet.

11. Waschkabine für zweirädrige Fahrzeuge nach einem der vorstehenden Ansprüche 1 bis 8, **dadurch gekennzeichnet, dass** die Waschkammer (4) einen Satz Ultraviolettlichtlampen umfasst.

12. Waschkabine für zweirädrige Fahrzeuge nach einem der vorstehenden Ansprüche, **dadurch gekennzeichnet, dass** sie einen Niederdruckwasserkreislauf umfasst, der sich oben auf der Waschkammer (4) befindet, um das Fahrzeug abzuspülen.

13. Waschkabine für zweirädrige Fahrzeuge nach einem der vorstehenden Ansprüche, **dadurch gekennzeichnet, dass** sie einen Niederdruckkreislauf für die Aufbringung von Entfettungsflüssigkeit innerhalb der Waschkammer (4) umfasst.

## Revendications

1. Cabine de lavage pour véhicules à deux roues, comprenant :
- une enceinte intérieure (3) qui divise l'espace intérieur en une chambre de lavage (4) et une armoire de machines (5) ; et
- une porte (20) pour accéder à la chambre de lavage (4) ;
- dans laquelle l'armoire de machines (5) comprend un circuit hydraulique haute pression et la chambre de lavage (4) comprend plusieurs turbines de lavage (6) ;
la cabine de lavage étant **caractérisée en ce que** :
les turbines de lavage (6) comprennent un corps central fixe (6.1) relié à une extrémité au circuit hydraulique haute pression et, de l'autre, à une tête (6.2) qui tourne avec le passage de l'eau, qui comprend à son tour au moins deux bras tubulaires (6.3) se terminant par deux buses d'hélice d'eau (6.4), dans laquelle l'axe passant par le centre des buses d'hélice (6.4) forme avec l'axe de rotation de la tête (6.2) un angle α compris entre 2° et 20° ; et
- dans laquelle chacun des bras tubulaires (6.3) est formé par une première section fixée à la tête (6.2) et par une seconde section se terminant au niveau de la buse d'hélice (6.4), dans laquelle les deux sections forment un angle l'une par rapport à l'autre ;
- dans laquelle l'angle formé par les première et seconde sections des bras tubulaires (6.3) est supérieur à 90°.

2. Cabine de lavage pour véhicules à deux roues selon la revendication 1, **caractérisée en ce que** la cabine présente une forme de prisme rectangulaire et inclut, à l'intérieur de sa chambre de lavage (4), des turbines de lavage (6) à la fois sur ses deux parois latérales les plus grandes et sur la paroi latérale la plus petite qui ne constitue pas la porte d'entrée (20).

3. Cabine de lavage pour véhicules à deux roues selon la revendication 2, **caractérisée en ce que** la chambre de lavage (4) comprend, au moins :
- deux turbines de lavage (6) sur la plus petite paroi latérale pour un nettoyage avant du véhicule ; et
- sur chacune des deux plus grandes parois latérales :
- deux turbines de lavage (6) en correspondance avec la zone où est située la partie avant du véhicule.
- une turbine de lavage (6) en correspondance avec la zone où est située la partie centrale du véhicule.
- deux turbines de lavage (6) en correspondance avec la zone où est située la partie arrière du véhicule.

4. Cabine de lavage pour véhicules à deux roues selon l'une quelconque des revendications précédentes, **caractérisée en ce que** l'armoire de machines (5) comprend en outre un système hydraulique basse pression relié au réseau d'alimentation en eau qui fournit de l'eau à un sous-système hydraulique basse pression à eau froide et à un sous-système hydraulique basse pression à eau chaude, dans laquelle chacun d'eux présente, respectivement, une électrovanne (8.9) pour permettre ou non le passage à une pompe haute pression (10) appartenant au système hydraulique haute pression.

5. Cabine de lavage pour véhicules à deux roues selon la revendication 4, **caractérisée en ce que** la pompe haute pression (10) est reliée à un collecteur haute pression (14) qui inclut des électrovannes haute pression (15) reliées aux turbines de lavage (6) et à un système de commande (17) permettant un fonctionnement total ou partiel desdites turbines de lavage (6) de sorte qu'il est possible de choisir les zones du véhicule qui seront soumises à un lavage.

6. Cabine de lavage pour véhicules à deux roues selon l'une quelconque des revendications précédentes, **caractérisée en ce que** l'angle α est compris entre 8° et 12°.

7. Cabine de lavage pour véhicules à deux roues selon la revendication 6, **caractérisée en ce que** l'angle α est de 10°.

8. Cabine de lavage pour véhicules à deux roues selon l'une quelconque des revendications précédentes, **caractérisée en ce que** l'armoire de machines (5) comprend une entrée d'air sous pression (18) reliée au collecteur haute pression (14) pour fournir de l'air de séchage à travers les turbines de lavage (6).

9. Cabine de lavage pour véhicules à deux roues selon la revendication 8, **caractérisée en ce que** l'armoire de machines (5) comprend un générateur d'ozone relié à l'entrée d'air sous pression (18) pour fournir ledit ozone à la chambre de lavage (4) à travers les turbines de lavage (6).

10. Cabine de lavage pour véhicules à deux roues selon l'une quelconque des revendications précédentes 1 à 8, **caractérisée en ce que** l'armoire de machines (5) comprend un générateur d'ozone relié à un circuit de dosage situé dans la chambre de lavage (4).

11. Cabine de lavage pour véhicules à deux-roues selon l'une quelconque des revendications précédentes 1 à 8, **caractérisée en ce que** la chambre de lavage (4) comprend un ensemble de lampes à lumière ultraviolette.

12. Cabine de lavage pour véhicules à deux roues selon l'une quelconque des revendications précédentes, **caractérisée en ce qu'**elle comprend un circuit d'eau basse pression situé sur le dessus de la chambre de lavage (4) pour rincer le véhicule.

13. Cabine de lavage pour véhicules à deux-roues selon l'une quelconque des revendications précédentes, **caractérisée en ce qu'**elle comprend un circuit basse pression pour l'application de liquide de dégraissage à l'intérieur de la chambre de lavage (4).
